# EUROPEAN PATENT SPECIFICATION

(11) **EP 3 916 393 B1**
(45) Date of publication and mention of the grant of the patent: **25.01.2023**
(21) Application number: 20176696.1
(22) Date of filing: 27.05.2020
(51) Int. Cl.: G01N 33/58

(54) **METHOD FOR DETECTION OF CELLS BY REPETITIVE STAINING AND DESTAINING**
VERFAHREN ZUM NACHWEIS VON ZELLEN DURCH WIEDERHOLTES FÄRBEN UND ENTFÄRBEN
PROCÉDÉ DE DÉTECTION DE CELLULES PAR COLORATION ET DÉCOLORATION RÉPÉTITIVES

(43) Date of publication of application: 01.12.2021
(73) Proprietor: Miltenyi Biotec B.V. & Co. KG, 51429 Bergisch Gladbach (DE)
(72) Inventor: FAUERBACH, Jonathan, 51429 Bergisch Gladbach (DE); BERNDT, Daniel, 51429 Bergisch Gladbach (DE); DOSE, Christian, D-51429 Bergisch Gladbach (DE); MEINEKE, Dirk, 51429 Bergisch Gladbach (DE)
(74) Representative: Kisters, Michael Marcus

(56) References cited:
- WO-A1-2016/144652
- WO-A1-2017/105927
- WO-A1-2018/009581
- WO-A1-2018/013389
- WO-A2-2010/115089
- US-A1- 2019 162 721
- ANGELA R DIXON ET AL: "Recent developments in multiplexing techniques for immunohistochemistry", EXPERT REVIEWS IN MOLECULAR DIAGNOSTICS, vol. 15, no. 9, 2015, pages 1171-1186, XP055604996,
- GEORGE GLASS ET AL: "Simple: A Sequential Immunoperoxidase Labeling and Erasing Method", JOURNAL OF HISTOCHEMISTRY AND CYTOCHEMISTRY, vol. 57, no. 10, 2009, pages 899-905, XP055676909,

## Description

### BACKGROUND

The present invention is directed to a process for detection or identification of target moieties or target cells from a cell sample by labelling the target moieties or target cells with a conjugate having a detection moiety and an antigen recognizing moiety, wherein after detecting the target moieties, the detection moieties are degraded by irradiation with light and thereby enabling subsequent labelling and detection.

Fluorescent moieties conjugated to one or more antibodies are commonly used for immunofluorescence analysis. A vast number of variants in view of antibodies, fluorescent moieties, flow cytometers, flow sorters, and fluorescence microscopes has been developed in the last two decades to enable specific detection and isolation of target cells.

It is known to utilize fluorescent moieties for isolated donor detection of target cells which can be removed or destroyed after detection. For example US7776562 discloses a method of reversible fluorescent labeling based on indirect, non-covalent labeling of target cells with reversible peptide/MHC-Multimers or Fab-streptamers.

In order to reduce the fluorescence radiation after detection, GB2372256 discloses a process to quench fluorescence radiation by providing a conjugate comprising a plurality of fluorescent moieties attached via a linker to an antibody. The high density of fluorescent moieties will quench the fluorescence signals. Furthermore, GB2372256 describes to enzymatic degrade the linker in order to release fluorescent moieties from the conjugate. The released fluorescent moieties are not subject to self-quenching, resulting in more intense fluorescence signals, i.e. in better resolution.

Elimination of the fluorescence signal is essential for immunofluorescence technologies based on sequentially staining specimen. These technologies have been shown to provide a higher multiplexing potential compared to standard procedures using simultaneously labeling and detection. However, these technologies are based on oxidative destruction of antibody conjugated fluorescent moieties by chemical bleaching procedures (US7741045B2, EP0810428B1 or DE10143757) or in the case of photobleaching based methods, the rate of bleaching is slower to the methods presented here. US2019/0162721A1 showed the increased bleaching of dyes by light after multimerization on branched PEG.

Conjugated polymers (CP) attached to small molecule dyes were also used for signal amplification and as bright fluorescent moieties as in US10126302B2 or US10481161B2. But no enhanced photobleaching is mentioned.

The main intention of the prior art was to provide dyes or conjugates comprising such dyes which emit fluorescence radiation as intense as possible i.e. with a maximum quantum yield. In order to provide reliable and reproducible signals, the dyes are designed to be as stable as possible. While these properties are advantageous for cell detection and cell separation like a FACS process, they prevent the cells from being repeatedly stained and detected.

### OBJECT OF THE INVENTION

Accordingly, there is a need to establish a method for staining and destaining of target moieties labeled with bright fluorescent moieties wherein the staining process provides signals as bright as possible which can be completely removed during destaining as fast as possible. It was found, that upon an appropriate selection of certain conjugates and custom method development, a very efficient staining/destaining process can be achieved.

Objection of the invention is a method for detecting a target moiety in a sample of biological specimens by providing a conjugate with the general formula (I) 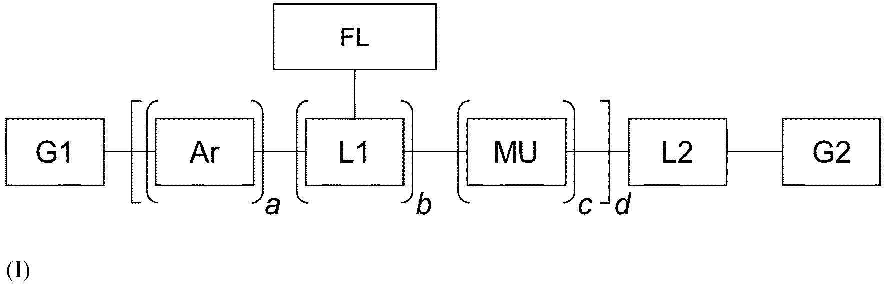
With Ar, MU and L1 as repeating units of a polymer
Ar is a aryl or heteroaryl group,
MU is a polymer modifying unit or band gap modifying unit that is evenly or randomly distributed along the polymer main chain,
L1 is an aryl or a heteroaryl group evenly or randomly distributed along the polymer,
L2 is an aryl or a heteroaryl group located on the ends of the polymer,
FL is a fluorescent moiety,
G1 and G2 stand for hydrogen, halogen or an antigen recognizing moiety, with the provision than at least one of G1 or G2 is an antigen recognizing moiety,
and
a is 10 to 100 mol %,
b is 0,1 to 50 mol %
c is 0 to 90 mol %
d is 1 to 10,000
with the provisio that a + b + c = 100 mol%
**characterized in that**

a) contacting the sample of biological specimens with at least one conjugate (I), thereby labeling the target moiety recognized by an antigen recognizing moiety with the conjugate (I);
b) exciting the labelled target moieties with light having a wavelength within the absorbance spectrum of the fluorescent moiety FL;
c) detecting the labelled target moieties by detecting the fluorescence radiation emitted by the fluorescent moiety FL and
d) degrading the fluorescent moiety FL of the labelled target moieties by irradiating the conjugate with light having a wavelength within the absorbance spectrum of fluorescent moiety FL for a time sufficient to deliver enough energy to reduce the fluorescence radiation emitted by the fluorescent moiety FL at least by 75 % of the initial fluorescence radiation.

Yet another object of the invention is the use of the method in fluorescence microscopy, flow cytometer, fluorescence spectroscopy, cell separation, pathology or histology.

### DETAILED DESCRIPTION

In the following, the conjugate according to formula (1) is referred to CP-FL with polymer backbone CP as defined as followed to which one or more fluorescent moieties FL are attached.

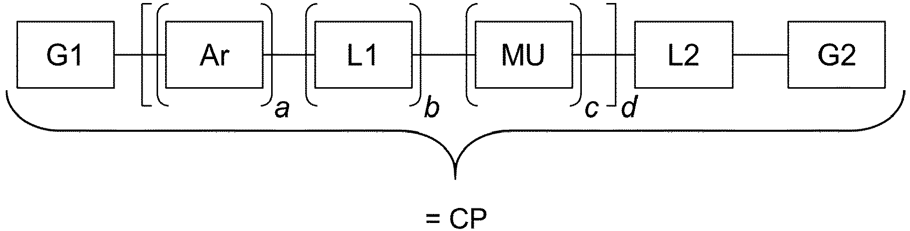

In the method of the invention, the sample is irradiated with light having a wavelength within the absorbance spectrum of the fluorescent moiety FL in order to reduce the fluorescence radiation emitted by the fluorescent moiety so much that any residual fluorescence radiation from a first staining cycle does not interfere with subsequent staining and detection cycles. In general, reduction by at least 75% of the initial fluorescence radiation is deemed sufficient, but in order to achieve a higher quality of detection i.e. to reduce background radiation not originating from the staining step of interest, it is preferred to reduce fluorescence radiation by at least by 85%, more preferred at least by 95 % and most preferred by at least 99%. While a reduction of 100% would be best, there is a trade-off with quenching quality and overall process duration.

In an alternative definition, degrading the fluorescent moiety FL attached to a conjugated polymer (CP) of the labelled target moieties is performed by irradiating the conjugate with light having a wavelength within the absorbance spectrum of fluorescent moiety FL or of CP or both (e.g. using white light) for a time sufficient to deliver enough energy to reduce the half-life of the fluorescence radiation emitted by the fluorescent moiety. The degradation rate given by the value of k from the mono-exponential decay fit analysis of the fluorescent moiety FL be at least 1.02 and up to 10.000.000 fold higher compared to the k obtained for the same fluorescent moiety non-conjugated to the conjugated polymer (CP).

Fluorescent moiety FL and antigen recognizing moiety can be bound covalently or quasi-covalently to CP. The terms "covalently or quasi-covalently" refers bonds between FL and CP and Y having a dissociation constant of greater or equal than 10⁻⁹ M.

The process of the invention may be performed in one or more sequences of the steps a) to d). After each sequence, the fluorescent moiety is degraded by irradiation with light. The terms "degrading", "quenching" or "bleaching" are used interchangeably herein, and should be understood to mean the diminution of fluorescence intensity from the labeled biological sample, as result of an alteration of the fluorophore by radiation. For example, "quenching" or "bleaching" of the fluorescent moiety FL may be achieved by oxidation initiated by the radiation and/or by cleaving the fluorescent moiety FL from CP and removing the unbound fluorescent moiety from the labelled target by washing.

The bleaching system used in the present invention may be provided with more than one light sources emitting radiation of different wavelengths. For example the bleaching system may be provided with 1-5 light sources which have a combined emission spectrum in the range of 350 - 850 nm, preferable 400 - 650 nm. The emission of the light sources may optically combined to irradiate the sample simultaneously or subsequently. For example, the bleaching system may be provided with four light sources emitting in the ranges 380 - 410 (violet), 450 - 500 nm (blue), 520 - 560 nm (green) and 630 - 650 nm (red). In another embodiment only one light source is provided, emitting light in the range 200 - 1000 nm (white light), preferable 350 - 850 nm, and most preferable 400 - 650 nm. The advantage of separate light sources is that the sample is exposed to radiation only necessary to bleach (eliminate) the fluorescence dye thereby avoiding unnecessary exposure of the sample to radiation with other wavelengths. The radiation of the separate light sources may be combined by appropriate devices like mirrors or optical waveguide like optical fiber.

After and/or before each sequence, a washing step may be performed to remove unwanted material like unbound conjugates moieties and/or unbound fluorescent moieties FL from the sample.

The bleaching process as described may be further enhanced by adding oxidative agents. Oxidative agents may be for example O₂, H₂O₂, peroxides or DMSO. The oxidative agents added may generate the active oxidative species, which, calculated as O, should be present in concentrations of 0.1 to 5 ppm, preferable 2 to 5 ppm.

### Target Moiety

The target moiety to be detected with the method of the invention can be on any biological specimen, like tissues slices, cell aggregates, suspension cells, or adherent cells. The cells may be living or dead. Preferable, target moieties are antigens expressed intracellular or extracellular on biological specimen like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo Sapiens).

### Fluorescent Moiety FL

Suitable fluorescent moieties FL are those known from the art of immunofluorescence technologies, e.g., flowcytometry or fluorescence microscopy. In the method of the invention, the target moiety labelled with the conjugate is detected by exciting the CP backbone or the the fluorescent moiety FL or both and detecting the resulting emission (photoluminescence) of FL or CP.

Useful fluorescent moieties FL might be protein based, such as phycobiliprotein, small organic molecule dyes, such as xanthenes, like fluorescein, or rhodamines, cyanines, oxazines, coumarins, acridines, oxadiazoles, pyrenes, pyrromethenes, pyridyloxazole or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

In another embodiment of the invention the target labelled with the conjugate is not detected by radiation emission, but by absorption of UV, visible light, or NIR radiation. Suitable light-absorbing detection moieties are light absorbing dyes without fluorescence emission, such as small organic molecule quencher dyes like N-aryl rhodamines, azo dyes, and stilbenes. In another embodiment, the light-absorbing fluorescent moieties FL can be irradiated by pulsed laser light, generating an photoacoustic signal.

In a variant of the invention, the fluorophore FL is substituted with one more water solubility imparting substituents selected from the group consisting of sulfonates, phosphonates, phosphates, polyethers, sulfonamides and carbonates. It is particularly advantageous to use fluorescent moieties with sulfonate substituents, such as dyes of the Alexa Fluor family provided by Thermo Fisher Scientific Inc. The degree of sulfonate substitution per fluorophore may be 2 or more, i.e., for rhodamine dyes or cyanine dyes.

Suitable commercial available fluorescent moieties may be purchased from the product line " Vio " from Miltenyi Biotec BV & Co. KG, or FITC, or Promofluor, or Alexa Dyes and/or Bodipy dyes from Thermofisher, or Cyanines from Lumiprobe or DY^{™} Fluorophore from Dyomics GmbH or Star Dyes from Abberior GmbH.

### Antigen recognizing moiety

The term "antigen recognizing moiety" refers to any kind of antibody, fragmented antibody or fragmented antibody derivatives, directed against the target moietiesexpressed on the biological specimens, like antigens expressed intracellular or extracellular on cells. The term relates to fully intact antibodies, fragmented antibody or fragmented antibody derivatives, e. g., Fab, Fab', F(ab')2, sdAb, scFv, di-scFv, nanobodies. Such fragmented antibody derivatives may be synthesized by recombinant procedures including covalent and non - covalent conjugates containing these kind of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cellsurface molecules.

The conjugate used in the method of the invention may comprise up to 100, preferable 1-20 antigen recognizing moieties Y. The interaction of the antigen recognizing moiety with the target antigen can be of high or low affinity. Binding interactions of a single low - affinity antigen recognizing moiety is too low to provide a stable bond with the antigen. Low-affinity antigen recognizing moieties can be multimerized by conjugation to the enzymatically degradable spacer to furnish high avidity. When the spacer is enzymatically cleaved, the low-affinity antigen recognizing moieties will be monomerized which results in a complete removal of the fluorescent marker.

Preferable, the term "Antigen recognizing moiety" refers to an antibody directed against antigen expressed by the biological specimens (target cells) intracellular, like IL2, FoxP3, CD154, or extracellular, like CD19, CD3, CD14, CD4, CD, CD25, CD34, CD56, and CD133. The antigen recognizing moieties G1, G2, especially antibodies, can be coupled to CP through side chain amino or sulfhydryl groups. In some cases the glycosidic side chain of the antibody can be oxidized by periodate resulting in aldehyde functional groups.

The antigen recognizing moiety can be covalently or non-covalently coupled. Methods for covalent or non-covalent conjugation are known by persons skilled in the art and the same as mentioned for conjugation of the fluorescent marker.

The method of the invention is especially useful for detection and/or isolation of specific cell types from complex mixtures and may comprise more than one sequentialsequences of the steps a) - d). The method may use a variety of combinations of conjugates. For example, a conjugate may comprise antibodies specific for two different epitopes, like two different anti-CD34 antibodies. Different antigens may be addressed with different conjugates comprising different antibodies, for example, anti-CD4 and anti-CD8 for differentiation between two distinct T-cell-populations or anti-CD4 and anti-CD25 for determination of different cell subpopulations like regulatory T-cells.

### Cell Detection Methods

Targets labelled with the conjugate are detected by exciting either the fluorescent moiety FL or the backbone CP and analysing the resulting fluorescence signal. The wavelength of the excitation is usually selected according to the absorption maximum of the fluorescent moiety FL or CP and provided by LASER or LED sources as known in the art. If several different detection moieties FL are used for multiple colour/parameter detection, care should be taken to select fluorescent moieties having not overlapping absorption spectra, at least not overlapping absorption maxima. In case of fluorescent moieties the targets may be detected, e.g., under a fluorescence microscope, in a flow cytometer, a spectrofluorometer, or a fluorescence scanner. Light emitted by chemiluminescence can be detected by similar instrumentation omitting the excitation.

### Use of the Method

The method of the invention can be used for various applications in research, diagnostics and cell therapy, like in fluorescence microscopy, flow cytometer, fluorescence spectroscopy, cell separation, pathology or histology.

In a first variant of the invention, biological specimens like cells are detected for counting purposes i.e. to establish the amount of cells from a sample having a certain set of antigens recognized by the antigen recognizing moieties of the conjugate. In another variant, the biological specimens detected by the conjugate in step c) are separated from the sample by optical means, electrostatic forces, piezoelectric forces, mechanical separation or acoustic means. For this purpose, the biological specimens detected by the conjugate in step d) are separated from the sample according to their detection signal to one or more populations simultaneously or subsequent before performing step d) by optical means, electrostatic forces, piezoelectric forces, mechanical separation or acoustic means.

In another variant of the invention, the location of the target moieties like antigens on the biological specimens recognized by the antigen recognizing moieties of the conjugate is determined. Such techniques are known as "Multi Epitope Ligand Cartography", "Chip-based Cytometry" or "Multiomyx" and are described, for example, in EP0810428, EP1181525, EP 1136822 or EP1224472. In this technology, cells are immobilized and contacted with antibodies coupled to fluorescent moiety. The antibodies are recognized by the respective antigens on the biological specimen (for example on a cell surfacd) and after removing the unbound marker and exciting the furescentieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties.

The location of the target moieties is achieved by a digital imaging device with a sufficient resolution and sensitivity in for the wavelength of the fluorescence radiation, The digital imaging device may be used with or without optical enlargement for example with a fluorescence microscope. The resulting images are stored on an appropriate storing device like a hard drive, for example in RAW, TIF, JPEG, or HDF5 format.

In order to detect different antigens, different antibody-conjugates having the same or different fluorescent moiety or antigen recognizing moiety can be provided. Since the parallel detection of fluorescence emission with different wavelengths is limited, the antibody-fluorochrome conjugates are utilized sequentially individually or in small groups (2-10) after the other.

In yet another variant of the method according to the invention, the biological specimens especially suspension cells of the sample are immobilized by trapping in microcavities or by adherence.

In general, the method of the invention can be performed in several variants. For example, the conjugate not recognized by a target moiety can be removed by washing for example with buffer before the target moiety labelled with the conjugate is detected.

In a variant of the invention, at least two conjugates are provided simultaneously or in subsequent staining sequences, wherein each antigen recognizing moiety recognizes different antigens. In an alternative variant, at least two conjugates can be provided to the sample simultaneously or in subsequent staining sequences, . In both cases, the labelled target moieties can be detected simultaneously or sequentially.

### EXAMPLES

The following compounds were investigated for their absorption behavior :
CP is 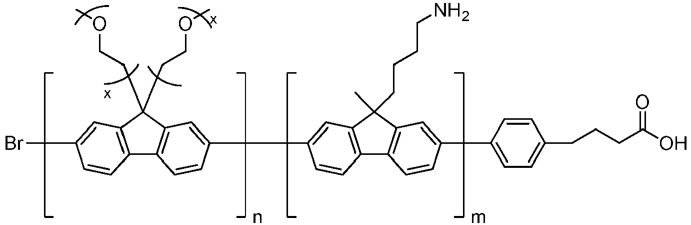
with n = 0.9,
m = 0.1 and
x = 11,
and CP-FL is 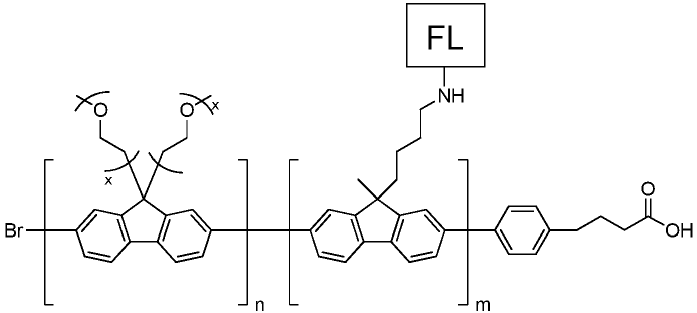
with n = 0.9,
m = 0.1 and
x = 11.
With FL= fluorescein rhodamines, cyanines or carbopyronine

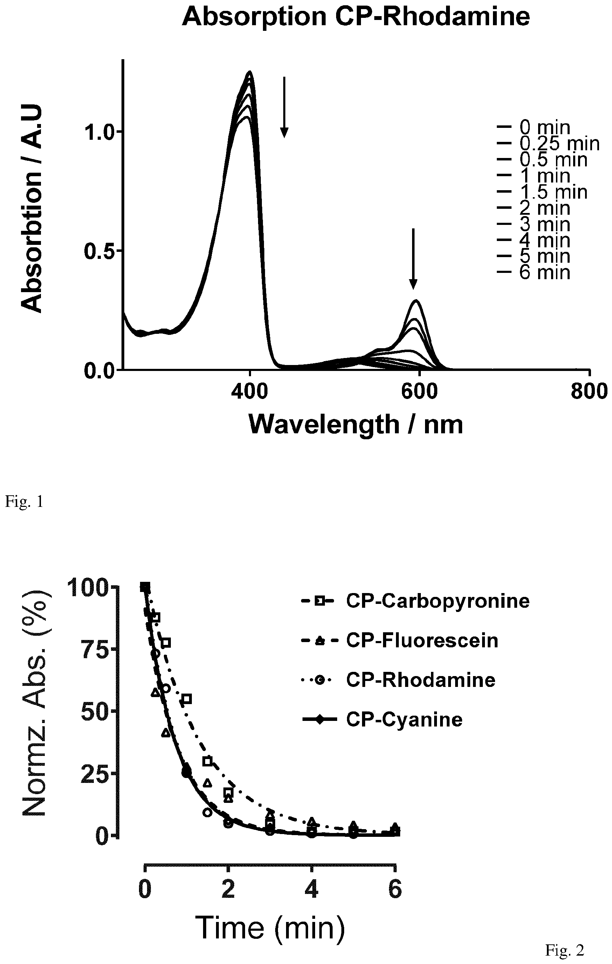

In order to illustrate the general kinetics involved in photodegradation, FIG. 1 shows a schematic curve obtained for photodegradation of a xanthene dye by light fitted with a mono - exponential decay curve such as *f*(x)= y0^{∗}exp(-*k*^{∗}x), where tau = 1/k and t_{1/2} = tau^{∗}ln(2) is the half-life. To measure the kinetics of photodegradation, organic fluorophores (i. e. coumarines, xanthenes, rhodamines, cyanines, among others) were either dissolved in DMSO and diluted in PBS or directly dissolved in PBS such that the concentration was adjusted to obtain an absorbance at their respective maxima of ca. 0.3 A.U. with a path-length of 1,00 cm. This way all solutions are normalized by their absorbance and comparable. Then the solution was placed inside a 3 way window fluorescence quartz cuvette with low head space and an air-tight top to avoid evaporation and sample concentration. The sample in the cuvette was then irradiated for fixed amount of times and both the absorbance and mission spectra were recorded. Intensity values and their maxima were used to plot vs. irradiation time and then a mathematical fit to mono-exponential decays was performed by appropriate computer software to obtain the curves as one shown in FIG. 1, with an absorption high absorption of the CP at roughly 400 nm and a redshifted absorption of the FL. Characteristic decay times (k) were used to calculate half-life among other parameters.

FIG. 2. shows photodegrading decay curves for a series of dyes belonging to a different chemical classification according to the nature of their chromophore (i.e. fluorescein rhodamines, cyanines, carbopyronine) where each dye is covalently attached to a CP moiety. The data shown in FIG. 2 show that all dye classes are sensitive to photo-degradation when they are attached to a CP as defined below and the rate of photodegradation is higher for constructs of this application than for FL itself.

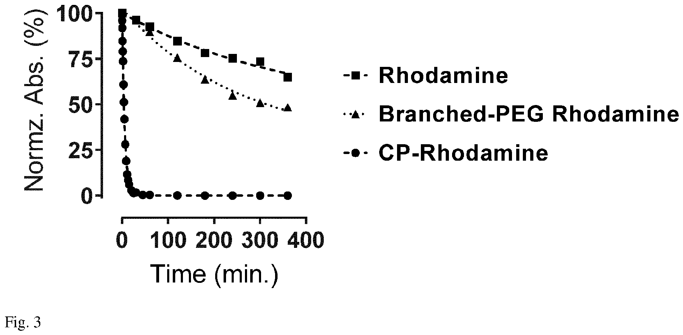

FIG. 3 shows photodegradation curves obtained for a rhodamine dye under three different conditions: i) not bound to anything and free in solution, ii) attached to branched PEG as described in US2019/0162721A1 iii) covalently attached to a linear CP (according to this invention).

The results of table 1 show the different constructs of CP-FL compared to small molecule moieties (FL) show an increase in the bleaching constant K for the different constructs by a factor of 94 (Fluorescein), 22.5 (Rhodamine) and 5.2 (Cyanine), while the half-life of the fluorophores is reduced by a factor of 38 (Fluorescein), 156 (Rhodamine), 98 (Cyanine).

As shown in Fig. 3 the herein presented invention (e.g. CP-Rhodamine ) shows an increase in the bleaching constant of factor 46 over the constructs from US2019/0162721A1 (Branched PEG Rhodamine) using a multimerization of FL on branched PEG as the state of the art. This higher bleaching constant leads to shorter bleaching times and less background e.g. in cycling imaging applications.

**Table 1: Comparison of bleaching behavior of small molecule dyes and small molecule dyes as the FL part bound to CP**

| **Dye** | **Fluorescein** | | **Rhodamine** | | **Cyanine** | |
|---|---|---|---|---|---|---|
| Construct | CP-FL | FL | CP-FL | FL | CP-FL | FL |
| k [min⁻¹] | 1.622 | 152.1 | 0.1789 | 4.02 | 1.417 | 5.16 |
| τ [min] | 0.43 | 16.2 | 3.87 | 606 | 0.49 | 48 |

## Claims

1. Method for detecting a target moiety in a sample of biological specimens by providing a conjugate with the general formula (I) 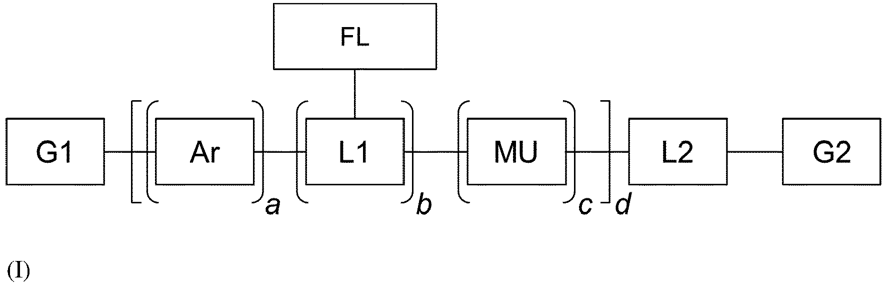
With Ar, MU and L1 as repeating units of a polymer
Ar is a aryl or heteroaryl group,
MU is a polymer modifying unit or band gap modifying unit that is evenly or randomly distributed along the polymer main chain,
L1 is an aryl or a heteroaryl group evenly or randomly distributed along the polymer,
L2 is an aryl or a heteroaryl group located on the ends of the polymer,
FL is a fluorescent moiety,
G1 and G2 stand for hydrogen, halogen or an antigen recognizing moiety, with the provision than at least one of G1 or G2 is an antigen recognizing moiety,
and
a is 10 to 100 mol %,
b is 0,1 to 50 mol %
c is 0 to 90 mol %
d is 1 to 10,000
with the provisio that a + b + c = 100 mol%
**characterized in**
a) contacting the sample of biological specimens with at least one conjugate (I), thereby labeling the target moiety recognized by an antigen recognizing moiety with the conjugate (I);
b) exciting the labelled target moieties with light having a wavelength within the absorbance spectrum of the fluorescent moiety FL;
c) detecting the labelled target moieties by detecting the fluorescence radiation emitted by the fluorescent moiety FL and
d) degrading the fluorescent moiety FL of the labelled target moieties by irradiating the conjugate with light having a wavelength within the absorbance spectrum of fluorescent moiety FL for a time sufficient to deliver enough energy to reduce the fluorescence radiation emitted by the fluorescent moiety FL at least by 75 % of the initial fluorescence radiation.

2. The method according to claim 1, **characterized in that** Ar is a aryl or heteroaryl group repeat unit substituted with a non-ionic side chain selected from the groups of an ethylene glycol oligomer side, dextran or glycerol.

3. The method according to claim 1 or 2, **characterized in that** MU is a polymer modifying unit or band gap modifying unit that is evenly or randomly distributed along the polymer main chain and is optionally substituted with one or more optionally substituted substituents selected from halogen, hydroxyl, C1-C12 alkyl, C2-C12 alkene, C2-C12 alkyne, C3-C12 cycloalkyl, C1-C12 haloalkyl, C1-C12 alkoxy, C2-C18 (hetero)aryloxy, C2-C18 (hetero) arylamino, a C2-C18 (hetero)aryl group and (CH2)x',(OCH2CH2)y'OCH3 where x' is independently an integer from 0-20 and y' is independently an integer from 0 to 50.

4. The method according to any of the claims 1 to 3, **characterized in that** L1 is an aryl or a heteroaryl group evenly or randomly distributed along the polymer main chain and is substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) an attached conjugated organic dye as acceptor dye, or iii) a biomolecule.

5. The method according to any of the claims 1 to 4, **characterized in that** L2 is an aryl or a heteroaryl group located on the ends of the polymer main chain and is substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) an attached organic dye as acceptor dye, or iii) a biomolecule.

6. The method according to any of the claims 1 to 5, **characterized in that** FL is selected from the group consisting of Fluorescein, Fluorescein-Derivatives, Rhodamine, Tetramethylrhodamine, Silicon-Rhodamine (SiR), Coumarines, Resorufines, Pyrenes, Anthracenes, Phenylenes, Phthalocyanines, Cyanines, Xanthenes, Amidopyrylium-Fluorophores, Oxazine, Quadrain-Farbstoffe, Carbopyronine, 7-Nitrobenz-2-Oxa-1,3-Diazol (NBD) Fluorophore, BODIPY^{™} Fluorophores (Molecular Probes, Inc.), ALEXA^{™} Fluorophore (Molecular Probes, Inc.), DY^{™} Fluorophores (Dyomics GmbH), Benzopyrylium Fluorophores, Benzopyrylium-Polymethine Fluorophores, Lanthanid-Chelate, Metalloporhyrines, Rhodol dyes, Carborhodol dyes, Naphthalimides und Porphyrines.

7. The method according to any of the claims 1 to 6, **characterized in that** G1 and G2 are both independently chosen from the group consisting of hydrogen, halogen or an antigen recognizing moiety at least one is biomolecule selected from the group onsisting of an antibody, an fragmented antibody, an fragmented antibody derivative, peptide/MHC-complexes, receptors for cell adhesion or costimulatory molecules, receptor ligands, antigens, hapten binders, avidin, streptavidin, travidin, aptamers, primers and ligase substrates, peptide/MHC complexe targeting TCR molecules, cell adhesion receptor molecules, receptors for costimulatory molecules or artificial engineered binding molecules.

8. The method according to any of the claims 1 to 7, **characterized in** providing a conjugate according to general formula (II) 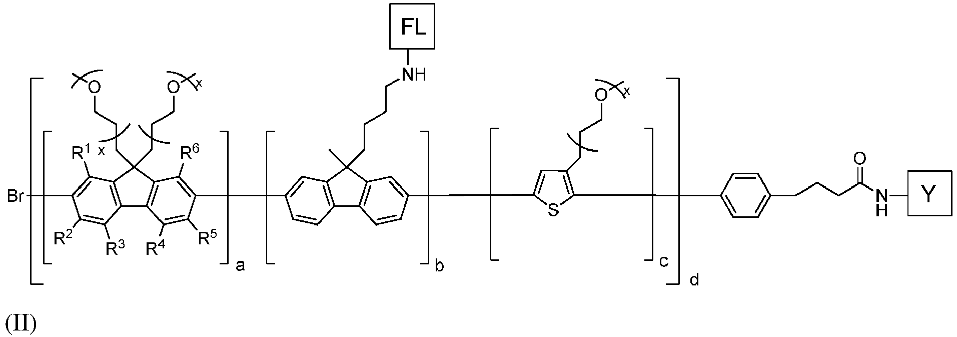 With
R¹, R², R³, R⁴, R⁵, R⁶, each same or independently = H, SO₂CF₃, SO₂R^{a}, CF₃, CCl₃, CN, SO₃H, NO₂, NR^{a}R^{b}R^{c+}, CHO, COR^{a}, CO₂R^{a}, COCl, CONR^{a}R^{b}, F, Cl, Br, I, R^{a}, OR^{a}, SR^{a}, OCOR^{a}, NR^{a}R^{b}, NHCOR^{a}, CCR^{a}, aryl-, heteroaryl-, C₆H₄OR^{a} or C₆H₄NR^{a}R^{b}, with R^{a-c} independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue, or two residues both as part of a cycloalkyl- or heterocycloalkyl ring system and each residue is made of 1 to 100 atoms.
x is an integer between 1 and 100,
y is an integer between 0 and 100,
a is 10 to 100 mol %,
b is 0,1 to 50 mol %
c is 0 to 90 mol %
d is 1 to 10,000
with the provisio that a + b + c = 100 mol%

9. The method according to any of the claims 1 to 8, wherein the fluorescent moiety FL of the labelled target moieties is further degraded by adding oxidative agents.

10. Use of the method of any of the claims 1 to 8, in fluorescence microscopy, flow cytometer, fluorescence spectroscopy, cell separation, pathology or histology.

## Patentansprüche

1. Verfahren zum Nachweisen einer Zielkomponente in einer Probe von biologischen Präparaten durch Bereitstellen eines Konjugats mit der allgemeinen Formel (I) 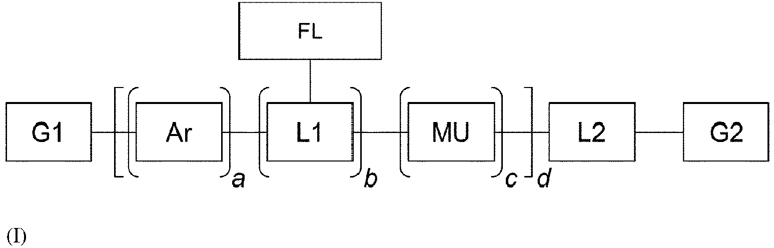 wobei Ar, MU und L1 Wiederholungseinheiten eines Polymers sind,
Ar eine Aryl- oder Heteroarylgruppe ist,
MU eine polymermodifizierende Einheit oder eine bandlückenmodifizierende Einheit ist, die gleichmäßig oder regellos entlang der Polymerhauptkette verteilt ist,
L1 eine Aryl- oder Heteroarylgruppe ist, die gleichmäßig oder regellos entlang des Polymers verteilt ist,
L2 eine Aryl- oder Heteroarylgruppe ist, die sich an den Enden des Polymers befindet,
FL eine fluoreszierende Komponente ist,
G1 und G2 für Wasserstoff, Halogen oder einen antigenerkennenden Rest stehen, mit der Maßgabe, dass mindestens einer von G1 oder G2 ein antigenerkennender Rest ist,
und
a 10 bis 100 Mol-% beträgt,
b 0,1 bis 50 Mol-% beträgt,
c 0 bis 90 Mol-% beträgt,
d 1 bis 10.000 beträgt,
mit der Maßgabe, dass a + b + c = 100 Mol-% sind, **gekennzeichnet durch das**
a) Inkontaktbringen der Probe von biologischen Präparaten mit mindestens einem Konjugat (I), wodurch die von einer antigenerkennenden Komponente erkannte Zielkomponente mit dem Konjugat (I) markiert wird;
b) Anregen der markierten Zielkomponenten mit Licht, das eine Wellenlänge innerhalb des Absorptionsspektrums der fluoreszierenden Komponente FL aufweist;
c) Nachweisen der markierten Zielkomponenten durch Nachweisen der von der fluoreszierenden Komponente FL emittierten Fluoreszenzstrahlung und
d) Abbauen der fluoreszierenden Komponente FL der markierten Zielkomponenten durch Bestrahlen des Konjugats mit Licht mit einer Wellenlänge innerhalb des Absorptionsspektrums der fluoreszierenden Komponente FL für eine Zeit, die ausreicht, um genügend Energie zu liefern, um die von der fluoreszierenden Komponente FL emittierte Fluoreszenzstrahlung um mindestens 75 % der ursprünglichen Fluoreszenzstrahlung zu reduzieren.

2. Verfahren nach Anspruch 1, **dadurch gekennzeichnet, dass** Ar eine Aryl- oder Heteroarylgruppen-Wiederholungseinheit ist, die mit einer nichtionischen Seitenkette substituiert ist, die aus den Gruppen einer Ethylenglykol-Oligomerseite, Dextran oder Glycerin ausgewählt ist.

3. Verfahren nach Anspruch 1 oder 2, **dadurch gekennzeichnet, dass** MU eine polymermodifizierende Einheit oder eine bandlückenmodifizierende Einheit ist, die gleichmäßig oder regellos entlang der Polymerhauptkette verteilt ist und gegebenenfalls mit einem oder mehreren gegebenenfalls substituierten Substituenten substituiert ist, ausgewählt aus Halogen, Hydroxyl, C1-C12-Alkyl, C2-C12-Alken, C2-C12-Alkin, C3-C12-Cycloalkyl, C1-C12-Halogenalkyl, C1-C12-Alkoxy, C2-C18-(Hetero)aryloxy, C2-C18-(Hetero)arylamino, einer C2-C18-(Hetero)arylgruppe und (CH2)x', (OCH2CH2)y'OCH3, wobei x' unabhängig für eine ganze Zahl von 0 bis 20 steht und y' unabhängig für eine ganze Zahl von 0 bis 50 steht.

4. Verfahren nach einem der Ansprüche 1 bis 3, **dadurch gekennzeichnet, dass** L1 eine Aryl- oder eine Heteroarylgruppe ist, die gleichmäßig oder regellos entlang der Polymerhauptkette verteilt ist und mit einer oder mehreren Seitenketten substituiert ist, die enden mit: i) einer funktionellen Gruppe, ausgewählt aus Amin, Carbamat, Carbonsäure, Carboxylat, Maleimid, aktiviertem Ester, N-Hydroxysuccinimidyl, Hydrazin, Hydrazid, Hydrazin, Azid, Alkin, Aldehyd, Thiol und geschützten Gruppen davon zur Konjugation mit einem Molekül oder Biomolekül; oder ii) einem angehängten konjugierten organischen Farbstoff als Akzeptorfarbstoff, oder iii) einem Biomolekül.

5. Verfahren nach einem der Ansprüche 1 bis 4, **dadurch gekennzeichnet, dass** L2 eine Aryl- oder Heteroarylgruppe ist, die sich an den Enden der Polymerhauptkette befindet und mit einer oder mehreren Seitenketten substituiert ist, die die enden mit: i) einer funktionellen Gruppe, ausgewählt aus Amin, Carbamat, Carbonsäure, Carboxylat, Maleimid, aktiviertem Ester, N-Hydroxysuccinimidyl, Hydrazin, Hydrazid, Hydrazin, Azid, Alkin, Aldehyd, Thiol und geschützten Gruppen davon zur Konjugation mit einem Molekül oder Biomolekül; oder ii) einem angehängten organischen Farbstoff als Akzeptorfarbstoff, oder iii) einem Biomolekül.

6. Verfahren nach einem der Ansprüche 1 bis 5, **dadurch gekennzeichnet, dass** FL ausgewählt ist aus der Gruppe bestehend aus Fluorescein, Fluorescein-Derivaten, Rhodamin, Tetramethylrhodamin, Silicium-Rhodamin (SiR), Cumarinen, Resorufinen, Pyrenen, Anthracenen, Phenylenen, Phthalocyaninen, Cyaninen, Xanthenen, Amidopyrylium-Fluorophoren, Oxazin, Quadrain-Farbstoffen, Carbopyronin, 7-Nitrobenz-2-Oxa-1,3-Diazol (NBD) Fluorophor, BODIPY^{™}-Fluorophoren (Molecular Probes, Inc.), ALEXA^{™}-Fluorophor (Molecular Probes, Inc.), DY^{™}-Fluorophoren (Dyomics GmbH), Benzopyrylium-Fluorophoren, Benzopyrylium-Polymethin-Fluorophoren, Lanthanid-Chelat, Metalloporhyrinen, Rhodol-Farbstoffen, Carborhodol-Farbstoffen, Naphthalimiden und Porphyrinen.

7. Verfahren nach einem der Ansprüche 1 bis 6, **dadurch gekennzeichnet, dass** G1 und G2 beide unabhängig voneinander ausgewählt sind aus der Gruppe bestehend aus Wasserstoff, Halogen oder einer antigenerkennenden Komponente, wobei mindestens einer ein Biomolekül ist, ausgewählt aus der Gruppe bestehend aus einem Antikörper, einem fragmentierten Antikörper, einem fragmentierten Antikörperderivat, Peptid/MHC-Komplexen, Rezeptoren für Zelladhäsionsmoleküle oder costimulatorische Moleküle, Rezeptorliganden, Antigenen, Haptenbindern, Avidin, Streptavidin, Travidin, Aptameren, Primern und Ligasesubstraten, Peptid/MHC-Komplexen, die auf TCR-Moleküle zielen, Zelladhäsionsrezeptormolekülen, Rezeptoren für costimulatorische Moleküle oder künstlich hergestellten Bindungsmolekülen.

8. Verfahren nach einem der Ansprüche 1 bis 7, **dadurch gekennzeichnet, dass** ein Konjugat gemäß der allgemeinen Formel (II) bereitgestellt wird. 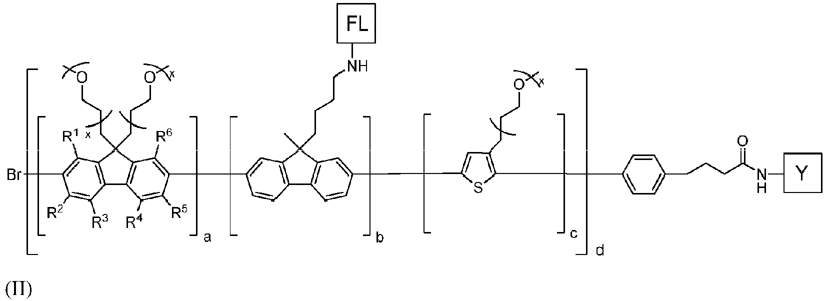 wobei
R¹, R², R³, R⁴, R⁵, R⁶, jeweils gleich oder unabhängig = H, SO₂CF₃, SO₂R^{a}, CF₃, CCl₃, CN, SO₃H, NO₂, NR^{a}R^{b}R^{c+}, CHO, COR^{a}, CO₂R^{a}, COCl, CONR^{a}R^{b}, F, Cl, Br, I, R^{a}, OR^{a}, SR^{a}, OCOR^{a}, NR^{a}R^{b}, NHCOR^{a}, CCR^{a}, Aryl-, Heteroaryl-, C₆H₄OR^{a} oder C₆H₄NR^{a}R^{b} sind, wobei R^{a-c} unabhängig Wasserstoff, Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl-oder ein Heteroaralkylrest oder zwei Reste ist, die beide Teil eines Cycloalkyl- oder Heterocycloalkylringsystems sind, und jeder Rest aus 1 bis 100 Atomen besteht,
x für eine ganze Zahl zwischen 1 und 100 steht,
y für eine ganze Zahl zwischen 0 und 100 steht,
a 10 bis 100 Mol-% beträgt,
b 0,1 bis 50 Mol-% beträgt,
c 0 bis 90 Mol-% beträgt,
d 1 bis 10.000 beträgt,
mit der Maßgabe, dass a + b + c = 100 Mol-% sind.

9. Verfahren nach einem der Ansprüche 1 bis 8, wobei die fluoreszierende Komponente FL der markierten Zielkomponenten durch Zugabe von Oxidationsmitteln weiter abgebaut wird.

10. Verwendung des Verfahrens nach einem der Ansprüche 1 bis 8, in der Fluoreszenzmikroskopie, Durchflusszytometrie, Fluoreszenzspektroskopie, Zellseparation, Pathologie oder Histologie.

## Revendications

1. Procédé de détection d'une fraction cible dans un échantillon de spécimens biologiques en fournissant un conjugué avec la formule générale (I) 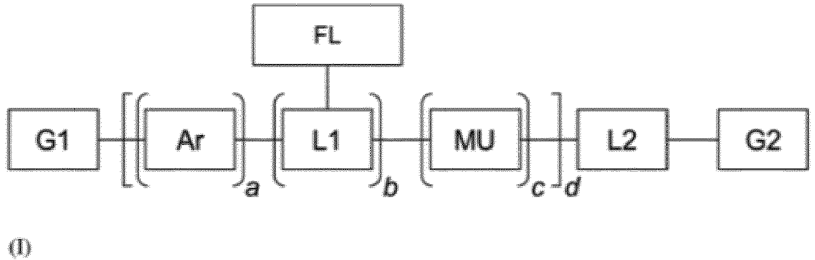
avec Ar, MU et L1 comme motifs répétés d'un polymère Ar est un groupe aryle ou hétéroaryle,
MU est une unité de modification de polymère ou une unité de modification d'espace libre de bande qui est distribuée uniformément ou au hasard le long de la chaîne principale de polymère,
L2 est un groupe aryle ou hétéroaryle situé aux extrémités du polymère,
FL est une fraction fluorescente,
G1 et G2 correspondent à un atome d'hydrogène, d'halogène ou à une fraction reconnaissant un antigène, à la condition qu'au moins un de G1 ou de G2 soit une fraction reconnaissant un antigène,
et
a est de 10 à 100 % en moles,
b est de 0,1 à 50 % en moles
c est de 0 à 90 % en moles
d est de 1 à 10 000
à la condition que a + b + c = 100 % en moles
**caractérisé par**
a) la mise en contact de l'échantillon de spécimens biologiques avec au moins un conjugué (I), marquant ainsi la fraction cible reconnue par une fraction reconnaissant un antigène avec le conjugué (I) ;
b) l'excitation des fractions cibles marquées avec de la lumière ayant une longueur d'onde dans le spectre d'absorption de la fraction fluorescente FL ;
c) la détection des fractions cibles marquées en détectant le rayonnement de fluorescence émis par la fraction fluorescente FL et
d) la dégradation de la fraction fluorescente FL des fractions cibles marquées en irradiant le conjugué avec de la lumière ayant une longueur d'onde dans le spectre d'absorption de la fraction fluorescente FL durant un temps suffisant pour délivrer assez d'énergie pour réduire le rayonnement de fluorescence émis par la fraction fluorescente FL au moins par 75 % du rayonnement de fluorescence initial.

2. Procédé selon la revendication 1, **caractérisé en ce qu'**Ar est un motif répété de groupe aryle ou hétéroaryle substitué avec une chaîne latérale non ionique choisie dans les groupes de côté d'oligomère d'éthylène glycol, de dextrane ou de glycérol.

3. Procédé selon la revendication 1 ou 2, **caractérisé en ce que** MU est une unité de modification de polymère ou une unité de modification d'espace libre de bande qui est distribuée uniformément ou au hasard le long de la chaîne principale du polymère et est éventuellement substituée avec un ou plusieurs substituants éventuellement substitués choisis parmi un atome d'halogène, un groupe hydroxyle, alkyle en C1 à C12, alcène en C2 à C12, alcyne en C2 à C12, cycloalkyle en C3 à C12, halogénoalkyle en C1 à C12, alcoxy en C1 à C12, (hétéro) aryloxy en C2 à C18, (hétéro) arylamino en C2 à C18, (hétéro) aryle en C2 à C18 et (CH2)x',(OCH2CH2)y'OCH3 où x' est indépendamment un nombre entier de 0 à 20 et y' est indépendamment un nombre entier de 0 à 50.

4. Procédé selon l'une quelconque des revendications 1 à 3, **caractérisé en ce que** L1 est un groupe aryle ou hétéroaryle distribué uniformément ou au hasard le long de la chaîne principale du polymère et est substitué avec une ou plusieurs chaînes pendantes terminées avec : i) un groupe fonctionnel choisi parmi une amine, un carbamate, l'acide carboxylique, un carboxylate, le maléimide, un ester activé, le N-hydroxysuccinimidyle, l'hydrazine, l'hydrazide, l'hydrazine, un azide, un alcyne, un aldéhyde, un thiol et leurs groupes protégés pour une conjugaison à une molécule ou une biomolécule ; ou ii) un colorant organique conjugué attaché comme colorant accepteur, ou iii) une biomolécule.

5. Procédé selon l'une quelconque des revendications 1 à 4, **caractérisé en ce que** L2 est un groupe aryle ou hétéroaryle situé sur les extrémités de la chaîne principale du polymère et est substitué avec une ou plusieurs chaînes pendantes terminées avec : i) un groupe fonctionnel choisi parmi une amine, un carbamate, l'acide carboxylique, un carboxylate, le maléimide, un ester activé, le N-hydroxysuccinimidyle, l'hydrazine, l'hydrazide, l'hydrazine, un azide, un alcyne, un aldéhyde, un thiol et leurs groupes protégés pour une conjugaison à une molécule ou une biomolécule ; ou ii) un colorant organique attaché comme colorant accepteur, ou iii) une biomolécule.

6. Procédé selon l'une quelconque des revendications 1 à 5, **caractérisé en ce que** FL est choisi dans le groupe constitué par la fluorescéine, les dérivés de fluorescéine, la rhodamine, la tétraméthylrhodamine, le silicium-rhodamine (SiR), les coumarines, les résorufines, les pyrènes, les anthracènes, les phénylènes, les phtalocyanines, les cyanines, les xanthènes, les fluorophores amydopyrilium, l'oxazine, la quadraïne-farbstoffe, la carbopyrosine, le fluorophore 7-nitrobenz-2-oxa-1,3-diazole (NBD), les fluorophores BODIPY^{™} (Molecular Probes, Inc.), le fluorophore ALEXA^{™} (Molecular Probes, Inc.), les fluorophores DY^{™} (Dyomics, GmbH), les fluorophores benzopyrilium, les fluorophores benzopyrilium-polyméthine, le lanthanide-chélate, les métalloporphyrines, les colorants rhodol, les colorants carborhodol, les naphtalimides et les porphyrines.

7. Procédé selon l'une quelconque des revendications 1 à 6, **caractérisé en ce que** G1 et G2 sont chacun indépendamment choisis dans le groupe constitué par un atome d'hydrogène, d'halogène ou une fraction reconnaissant un antigène au moins un est une biomolécule choisie dans le groupe constitué par un anticorps, un anticorps fragmenté, un dérivé d'anticorps fragmenté, des complexes peptide-MHC, les récepteurs pour l'adhésion cellulaire ou les molécules costimulatrices, les ligands de récepteur, les antigènes, les lieurs d'haptène, l'avidine, la streptavidine, la travidine, les aptamères, les amorces et les substrats de ligase, un complexe peptide/MHC ciblant des molécules TCR, les molécules de récepteur d'adhésion cellulaire, les récepteurs pour molécules costimulatrices ou les molécules de liaison modifiées artificielles.

8. Procédé selon l'une quelconque des revendications 1 à 7, **caractérisé par** la fourniture d'un conjugué selon la formule générale (II) 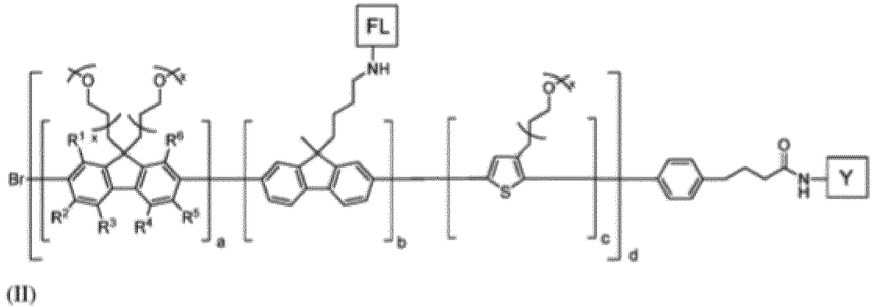 avec
R¹, R², R³, R⁴, R⁵, R⁶, chacun identique ou indépendamment = H, un groupe SO₂CF₃, SO₂R^{a}, CF₃, CCl₃, CN, SO₃H, NO₂, NR^{a}R^{b}R^{c+}, CHO, COR^{a}, CO₂R^{a}, COCl, CONR^{a}R^{b}, F, Cl, Br, I, R^{a}, OR^{a}, SR^{a}, OCOR^{a}, NR^{a}R^{b}, NHCOR^{a}, CCR^{a}, aryle-, hétéroaryle-, C₆H₄OR^{a} ou C₆H₄NR^{a}R^{b}, avec R^{a-c} indépendamment un atome d'hydrogène, un résidu alkyle-, alcényle-, alcinyle-, hétéroalkyle-, aryle-, hétéroaryle-, cycloalkyle-, alkylcycloalkyle-, hétéroalkylcycloalkyle-, hétérocycloalkyle-, aralkyle-ou hétéroaralkyle ou deux résidus tous les deux comme partie d'un système de cycle cycloalkyle- ou hétérocycloalkyle et chaque résidu est constitué de 1 à 100 atomes,
x est un nombre entier entre 1 et 100,
y est un nombre entier entre 0 et 100,
a est 10 à 100 % en moles,
b est 0,1 à 50 % en moles
c 'est 0 à 90 % en moles
d est 1 à 10 000
à la condition que a + b + c = 100 % en moles.

9. Procédé selon l'une quelconque des revendications 1 à 8, dans lequel la fraction fluorescente FL des fractions cibles marquées est en outre dégradée par addition d'agents oxydants.

10. Utilisation du procédé selon l'une quelconque des revendications 1 à 8, en microscopie par fluorescence, cytométrie de flux, spectroscopie par fluorescence, séparation cellulaire, pathologie ou histologie.
